# FASCICULE DE BREVET EUROPEEN

(11) **EP 3 393 580 B1**
(45) Date de publication et mention de la délivrance du brevet: **29.01.2020**
(21) Numéro de dépôt: 16825836.6
(22) Date de dépôt: 13.12.2016
(51) Int. Cl.: A61N 5/06

(54) **DISPOSITIF DE SUDATION PAR INFRAROUGE**
INFRAROT-SCHWEISSVORRICHTUNG
INFRARED SUDATION DEVICE

(30) Priorité: 22.12.2015 FR 1563065
(43) Date de publication de la demande: 31.10.2018
(73) Titulaire: Iyashi Dôme, 75012 Paris (FR)
(72) Inventeur: UEMURA, Shogoro, 1227 Carouge (CH)
(74) Mandataire: Fédit-Loriot
(86) Numéro de dépôt international: PCT/FR2016/053354
(87) Numéro de publication internationale: WO 2017/109332

(56) Documents cités:
- WO-A1-2015/059313
- CN-Y- 2 774 214
- CN-Y- 2 794 544
- FR-A1- 2 931 685
- JP-A- 2008 011 996

## Description

L'invention concerne de façon générale le domaine des appareils pour le traitement notamment de la peau par radiation et concerne plus particulièrement un dispositif de sudation par infrarouge et en particulier par infrarouge de type long.

Il est fait référence au document WO2015/059313.

L'utilisation d'ondes infrarouge en thérapie, en particulier des ondes du type infrarouge lointain aussi appelées IR-C, ou infrarouge long, dont la longueur d'onde se situe entre 3 µm et 1000 µm, permet une résonance du tissu musculaire augmentant certaines fonctions cellulaires générant une sudation particulièrement efficace pour l'amélioration de la qualité de la peau et de ses propriétés physiologiques. En particulier, l'énergie radiante fournie par un dispositif d'irradiation à infrarouge dans cette plage de longueurs d'onde procure une désintoxication en profondeur des couches supérieures du corps humain, avec un effet bénéfique sur le bien-être et sur la santé. En effet, à la différence d'un sauna traditionnel où la chaleur est transmise au corps en chauffant l'air ambiant, le dispositif d'irradiation à infrarouge permet de chauffer le corps directement par la chaleur produite par le rayonnement infrarouge. La chaleur diffusée par les infrarouges longs pénètre ainsi en profondeur dans la peau et permet d'éliminer efficacement par la sueur les toxines en excès, parmi lesquelles les métaux lourds toxiques auxquels ont pu être exposés les individus, tels que le strontium, le baryum, le nickel, le plomb, le molybdène, le tellure, le chrome, le cobalt, l'arsénique, le cadmium, l'aluminium et le cuivre. En effet, ces métaux lourds sont essentiellement excrétés par la sueur. Aussi, l'exposition au rayonnement infrarouge long permet d'augmenter l'élimination des métaux lourds. Il a pu être constaté au travers d'études visant à évaluer l'efficacité d'un dispositif d'irradiation à infrarouges longs que les propriétés physiologiques et biomécaniques de la peau d'un individu utilisant un tel dispositif, étaient améliorées (meilleure densité de la peau, meilleure élasticité cutanée et fermeté de la peau, atténuation des rides, etc.).

Le document de brevet US6,549,809 fait connaître un dispositif d'irradiation à infrarouge long du type comprenant un support destiné à recevoir un utilisateur en position allongée et une partie d'émission infrarouge pour irradier l'utilisateur, constituée de deux parties semi-cylindriques coulissant l'une sur l'autre et propres à recouvrir l'utilisateur allongé sur le support. Les parties semi-cylindriques sont équipées le long de leur surface interne de moyens d'émission infrarouge comprenant une couche chauffante à base de carbone noir (« black carbon » selon la terminologie anglo-saxonne) fixée sur la surface interne et apte à fournir un rayonnement dans les longueurs d'onde souhaitées dans le volume situé entre le support et les parties semi-cylindriques le recouvrant. Cependant, l'utilisation répétée du dispositif provoque une saturation de l'air en toxines éliminées par la sueur, qui est néfaste et non hygiénique.

Dans ce contexte, la présente invention a pour but de proposer un dispositif exempt de la limitation précédemment évoquée.

A cette fin, l'invention concerne un dispositif de sudation par infrarouge comprenant un élément de support s'étendant suivant un axe longitudinal destiné à recevoir un utilisateur en position allongée et un élément de couvercle de forme semi-cylindrique monté sur ledit élément de support de façon à délimiter un volume interne s'étendant dans la direction longitudinale dudit élément de support entre ledit élément de support et la surface interne dudit élément de couvercle, ladite surface interne dudit élément de couvercle étant recouverte au moins en partie d'une couche chauffante apte à émettre un rayonnement infrarouge lointain dans au moins une partie dudit volume interne, ledit dispositif étant caractérisé en ce qu'il comprend un logement de photocatalyseur, perméable au rayonnement infrarouge émis, supportant un photocatalyseur et étant disposé à proximité de la face interne de ladite couche chauffante, de façon à permettre l'activation dudit photocatalyseur à l'aide de l'énergie apportée par ledit rayonnement infrarouge émis.

Grâce à cet agencement, il est possible de décontaminer et dépolluer l'air présent au sein du volume interne soumis au rayonnement infrarouge pendant le déroulement d'une séance d'irradiation. En outre, ce processus de décontamination et de dépollution est particulièrement avantageux du fait qu'il repose sur l'utilisation d'un photocatalyseur activable par le rayonnement infrarouge lointain émis par la couche chauffante du dispositif de sudation et qui présente ainsi une performance photocatalytique suffisamment élevée même dans l'environnement du volume interne du dispositif avec peu ou pas du tout de lumière ultraviolette et ce, sans qu'il soit nécessaire de prévoir une source d'énergie dédiée à son activation.

De préférence, ledit photocatalyseur comprend un produit photocatalytique constitué d'un substrat métallique et/ou céramique sur la surface duquel est formée une couche de dioxyde de titane.

Avantageusement, ladite couche chauffante est apte à émettre un rayonnement infrarouge dans une plage de longueurs d'onde comprise entre 5 et 20 micromètres et plus avantageusement encore entre 8 et 14 micromètres.

Avantageusement, ledit logement de photocatalyseur comporte un châssis de forme sensiblement allongée, fixé entre deux extrémités intérieures, selon l'axe longitudinal, dudit élément de couvercle, de sorte à être maintenu en regard et à distance de ladite face interne de ladite couche chauffante recouvrant la surface interne dudit élément de couvercle.

De préférence, ledit châssis est monté à distance de ladite face interne de ladite couche chauffante en étant séparé par une lame d'air ayant une épaisseur égale à au moins 0,5 cm.

De préférence, l'axe dudit châssis s'étend colinéairement à l'axe longitudinal dans un plan perpendiculaire audit élément de support.

Avantageusement, ledit châssis présente une section transversale dont la forme suit le profil de ladite couche chauffante sur ladite face interne de ladite couche chauffante.

De préférence, ladite section transversale dudit châssis s'étend sur une portion limitée dudit profil de ladite couche chauffante.

Avantageusement, ledit châssis comprend un plateau inférieur ajouré comportant au moins une zone de réception destinée à recevoir ledit photocatalyseur et une tôle supérieure ajourée fermant ledit châssis en regard de ladite face interne de ladite couche chauffante.

Selon un mode de réalisation particulier, ledit logement de photocatalyseur intègre en outre un tissu apte à émettre des ondes électromagnétiques dans le domaine des infrarouges lointains dans sensiblement la même plage de longueurs d'onde que le rayonnement infrarouge lointain émis par ladite couche chauffante.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 est une vue schématique partielle de profil en perspective du dispositif de sudation par irradiation aux infrarouges lointains ;
- la Figure 2 est une vue de détail du côté intérieur d'un l'élément de couvercle du dispositif de sudation ;
- la Figure 3 est une vue schématique de dessus du logement de photocatalyseur illustré à la figure 2.

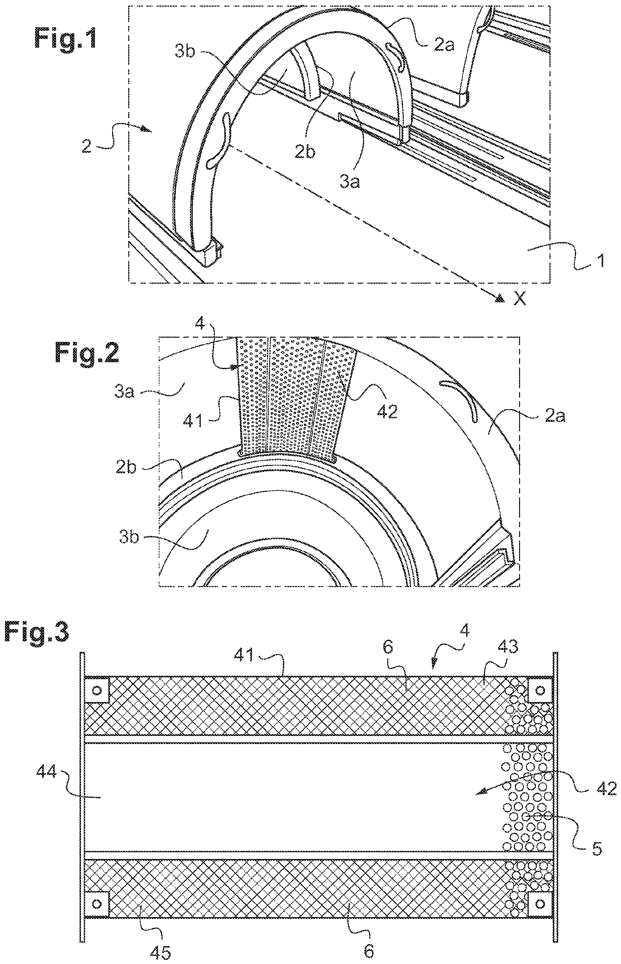

Le dispositif de sudation par infrarouge illustré à la figure 1 comprend un élément de support 1 s'étendant suivant un axe longitudinal X apte à recevoir un utilisateur notamment en position allongée. L'élément de support 1 est surmonté d'un élément de couvercle 2, de forme semi-cylindrique, qui est monté sur ledit élément de support 1 suivant l'axe longitudinal de celui-ci de façon à délimiter un volume interne s'étendant dans la direction longitudinale de l'élément de support 1, entre la face supérieure de l'élément de support 1 et une surface interne de l'élément de couvercle 2 disposée en vis-à-vis de la face supérieure de l'élément de support 1, lorsque l'élément de couvercle 2 est monté sur l'élément de support 1. L'élément de couvercle 2 permet ainsi de recouvrir totalement l'utilisateur allongé sur l'élément de support 1.

Selon un mode de réalisation préféré, l'élément de couvercle 2 comprend au moins une première partie semi-cylindrique 2a, dite partie semi-cylindrique supérieure, destinée à recouvrir la partie du corps de l'utilisateur située du côté de sa tête, et une deuxième partie semi-cylindrique 2b, dite partie semi-cylindrique inférieure, destinée à recouvrir la partie du corps de l'utilisateur située du côté opposé à la tête. La deuxième partie semi-cylindrique 2b présente par exemple un diamètre inférieur au diamètre de la première partie semi-cylindrique 2a et les première et deuxième parties semi-cylindriques 2a et 2b sont couplées ensemble de sorte qu'elles peuvent coulisser l'une par rapport à l'autre suivant la direction longitudinale X, par l'intermédiaire d'un système de coulissement, par exemple par l'intermédiaire de glissières agencées sur des parties latérales de l'élément de support 1, parallèlement à l'axe longitudinal X. En particulier la première partie cylindrique 2a peut coulisser suivant la direction longitudinale X sur la deuxième partie cylindrique 2b, de façon à s'ajuster à la taille de l'utilisateur. Une extrémité, non représentée, de la deuxième partie semi-cylindrique 2b de l'élément de couvercle 2 est obturée par une plaque semi-circulaire, permettant de fermer à cette extrémité le volume interne dans lequel est destiné à prendre place l'utilisateur.

Chaque partie semi-cylindrique 2a, 2b est équipée sur sa surface interne, c'est-à-dire la surface située du côté dudit volume interne, d'une couche chauffante, respectivement 3a, 3b, apte à émettre un rayonnement infrarouge, et plus particulièrement un rayonnement infrarouge lointain ou long, au sein du volume interne situé entre la face interne de la couche chauffante (c'est-à-dire la face en vis-à-vis de l'élément de support) et l'élément de support 1. La plaque semi-circulaire fermant le volume interne à une extrémité peut également être équipée sur sa surface interne d'une telle couche chauffante. Chaque couche chauffante 3a, 3b est montée sur la surface interne de la partie correspondante de l'élément de couvercle de façon à recouvrir sensiblement toute la surface interne en épousant le profil de cette surface interne. Ainsi, le profil de la couche chauffante permet d'obtenir une émission du rayonnement infrarouge répartie de façon optimale sur toute la surface du corps de l'utilisateur avec une zone de convergence du rayonnement émis située sensiblement le long de l'axe longitudinal et donc le long du corps de l'utilisateur allongé sous la couche chauffante intégrée à l'élément de couvercle.

Chaque couche chauffante 3a, 3b se présente par exemple sous la forme d'au moins une plaque de carbone, présentant une structure multicouche comprenant préférentiellement une couche à base de « black carbon », prise en sandwich entre deux feuilles en fibre de verre. La plaque est également munie de deux électrodes, disposées sur des bords opposés de la plaque, en contact avec la couche à base « black carbon » et susceptibles d'être alimentées par l'intermédiaire de moyens d'alimentation (non représentés) commandés par un contrôleur. Lorsque la couche à base de « black carbon » est alimentée par l'intermédiaire des électrodes, elle s'échauffe en émettant un rayonnement infrarouge long sur toute sa surface. Préférentiellement, on contrôle l'alimentation de la couche chauffante de sorte à émettre un rayonnement infrarouge long dans la plage de longueurs d'onde comprise entre 5 et 20 µm, préférentiellement encore, entre 8 et 14 µm. Un tel rayonnement reçu par le corps de l'utilisateur présente un pouvoir élevé de pénétration dans les tissus de la peau et permet de provoquer une élévation de la température de surface du corps de l'utilisateur. Une telle irradiation provoque une stimulation des tissus de la peau et notamment une stimulation des glandes sudoripares, qui favorise l'excrétion des produits chimiques toxiques pour le corps humain, en particulier les métaux lourds toxiques auxquels a pu être exposé l'utilisateur.

On prévoit en outre, conformément à l'invention, de dépolluer l'air au sein du volume interne délimité par l'élément de support 1 et son élément de couvercle 2, en associant un système de photocatalyse à l'émission du rayonnement infrarouge long dans ce volume interne, activable par ledit rayonnement, afin d'éviter une saturation de l'air en produits chimiques excrétés par la sudation de l'utilisateur soumis au rayonnement infrarouge long.

Pour ce faire, le système de photocatalyse mis en œuvre comprend un logement de photocatalyseur 4, perméable au rayonnement infrarouge émis, supportant un photocatalyseur 5 et étant disposé à proximité de la face interne de la couche chauffante 3a de la première partie semi-cylindrique 2a de l'élément de couvercle 2, de façon à permettre l'activation du photocatalyseur à l'aide de l'énergie apportée par le rayonnement infrarouge émis par la couche chauffante. On pourrait également prévoir d'équiper de la même manière la deuxième partie semi-cylindrique 2b de l'élément de couvercle 2 avec un tel système de photocatalyse.

Suivant le mode de réalisation illustré sur les figures, le logement 4 comporte un châssis 41, préférentiellement en métal, de forme sensiblement allongée, fixé entre deux extrémités intérieures, selon l'axe longitudinal X, de la première partie semi-cylindrique 2a de l'élément de couvercle, de sorte à être maintenu en regard et à une certaine distance de la face interne de la couche chauffante 3a. Le châssis 41 est disposé en regard de la face interne de la couche chauffante de sorte que son axe s'étende préférentiellement colinéairement à l'axe longitudinal X dans un plan perpendiculaire à l'élément de support. Autrement dit, le châssis 41 s'étend préférentiellement le long du sommet de l'élément de couvercle. Le châssis 41 comprend en outre une section transversale dont la forme suit sensiblement le profil de la couche chauffante 3a sur la face interne de la couche chauffante 3a en regard de laquelle il est disposé et cette section transversale s'étend de préférence sur une portion limitée du profil de la couche chauffante, de part et d'autre du sommet. Préférentiellement, le châssis 41 est monté à distance de la face interne de la couche chauffante 3a en étant séparé par une lame d'air ayant une épaisseur égale à au moins 0,5 cm.

Le châssis 41 comprend un plateau inférieur ajouré 42, formant une grille, dans lequel sont agencées des zones de réception séparées par des cloisons de séparation, par exemple trois zone de réception 43, 44, 45, qui s'étendent longitudinalement dans le châssis et dont au moins la zone de réception centrale 44 est destinée à recevoir le photocatalyseur.

Cette grille 42 est recouverte d'une tôle supérieure ajourée (non représentée) fermant le châssis en regard de la face interne de la couche chauffante 3a. Aussi, la structure de l'ensemble formant le châssis est perméable au rayonnement infrarouge long émis par la couche chauffante grâce aux zones ajourées et le passage du rayonnement infrarouge long émis par la couche chauffante 3a au travers des zones ajourées du châssis va permettre d'exciter le photocatalyseur 5 stocké à l'intérieur des zones de réception du châssis 41.

Dans un mode de réalisation particulier, le logement de photocatalyseur 4 intègre un tissu apte à émettre des ondes électromagnétiques en particulier dans le domaine des infrarouges lointains, dans sensiblement la même plage de longueurs d'onde que le rayonnement infrarouge émis par la couche chauffante. Ainsi, selon ce mode de réalisation particulier, les zones de réception latérale 43, 45, qui s'étendent de chaque côté de la zone de réception centrale 44 où est reçu le photocatalyseur 5, sont destinées à recevoir une couche de tissu 6 réalisé à base de fibres, notamment des fibres telles que décrites dans le document de brevet EP2072666, contenant du diamant nanométrique et un colloïde nanométrique de platine, le diamant nanométrique et le colloïde nanométrique de platine étant fixés auxdites fibres. De telles fibres présentent une excellente capacité à émettre un rayonnement infrarouge lointain. L'intégration d'un tel tissu au logement de photocatalyseur 4 permet d'amplifier le rayonnement infrarouge lointain émis au sein du volume interne du dispositif grâce à la capacité naturelle d'émission d'un tel rayonnement que présente ce tissu. Cet effet d'amplification est d'autant plus notable que l'action de ce tissu est favorisée par la chaleur produite par la couche chauffante, qui chauffe à une température de l'ordre de 55 à 70°C. En outre, le logement de photocatalyseur 4 forme une sorte d'écran devant la couche chauffante 3a à l'endroit où il est disposé, soit sensiblement le long du sommet de l'élément de couvercle selon l'exemple de réalisation, puisque les parties entre les zones ajourées du châssis nuisent à la bonne propagation vers le volume interne du rayonnement infrarouge lointain émis depuis le sommet de l'élément de couvercle. Aussi, l'intégration de la couche de tissu 6 au logement de photocatalyseur 4 permet de compenser cette relative perte de rayonnement infrarouge lointain émis par la couche chauffante au niveau du sommet de l'élément de couvercle et permet ainsi d'obtenir une répartition de l'émission du rayonnement infrarouge relativement uniformisée au sein du volume interne, en dépit de la présence du logement de photocatalyseur 4.

Le photocatalyseur 5 stocké dans le logement de photocatalyseur 4 et par exemple dans la zone de réception centrale à l'intérieur du châssis 41, comprend un produit photocatalytique, se présentant par exemple sous la forme de billes de faibles dimensions, préférentiellement de l'ordre de 15 à 20 mm de diamètre, revêtues d'un photocatalyseur. Chaque bille forme ainsi un agent photocatalyseur et est constituée plus précisément d'un substrat métallique et/ou céramique sur la surface duquel est formée une couche de dioxyde de titane appliquée suivant le procédé de fabrication décrit dans le brevet EP0980709. Cette technologie est connue sous la dénomination anglo-saxonne « PIP process », PIP étant un acronyme pour l'expression anglo-saxonne « Powder Impact Plating ».

Par agent photocatalyseur, on désigne un agent apte à détruire les différents polluants organiques présents dans l'air et ce, par réaction photocatalytique provoquée par l'irradiation de l'agent photocatalyseur par les rayons infrarouge longs émis par la couche chauffante 3a. Cette réaction chimique est bien connue sous le terme de photocatalyse et est avantageusement ici mise en œuvre pour le traitement et la dépollution de l'air compris dans le volume d'air entre l'élément de couvercle et l'élément de support au cours d'une séance de sudation par infrarouge long. Ainsi, les particules polluantes en suspension dans l'air et entrant au contact des surfaces des agents photocatalyseurs traitées avec le revêtement photocatalyseur, sont décomposées par la réaction photocatalytique.

## Revendications

1. Dispositif de sudation par infrarouge comprenant un élément de support (1) s'étendant suivant un axe longitudinal (X) destiné à recevoir un utilisateur en position allongée et un élément de couvercle (2a, 2b) de forme semi-cylindrique monté sur ledit élément de support (1) de façon à délimiter un volume interne s'étendant dans la direction longitudinale dudit élément de support entre ledit élément de support et la surface interne dudit élément de couvercle, ladite surface interne dudit élément de couvercle étant recouverte au moins en partie d'une couche chauffante (3a, 3b) apte à émettre un rayonnement infrarouge lointain dans au moins une partie dudit volume interne, ledit dispositif étant **caractérisé en ce qu'**il comprend un logement de photocatalyseur (4), perméable au rayonnement infrarouge émis, supportant un photocatalyseur (5) et étant disposé à proximité de la face interne de ladite couche chauffante (3a, 3b) de façon à permettre l'activation dudit photocatalyseur à l'aide de l'énergie apportée par ledit rayonnement infrarouge émis.

2. Dispositif selon la revendication 1, **caractérisé en ce que** ledit photocatalyseur (5) comprend un produit photocatalytique constitué d'un substrat métallique et/ou céramique sur la surface duquel est formée une couche de dioxyde de titane.

3. Dispositif selon l'une quelconque des revendications précédentes, **caractérisé en ce que** ladite couche chauffante (3a, 3b) est apte à émettre un rayonnement infrarouge dans une plage de longueurs d'onde comprise entre 5 et 20 micromètres, de préférence comprise entre 8 et 14 micromètres.

4. Dispositif selon l'une quelconque des revendications précédentes, **caractérisé en ce que** ledit logement de photocatalyseur (4) comporte un châssis (41) de forme sensiblement allongée, fixé entre deux extrémités intérieures, selon l'axe longitudinal, dudit élément de couvercle, de sorte à être maintenu en regard et à distance de ladite face interne de ladite couche chauffante (3a) recouvrant la surface interne dudit élément de couvercle (2a).

5. Dispositif selon la revendication 4, **caractérisé en ce que** ledit châssis (41) est monté à distance de ladite face interne de ladite couche chauffante en étant séparé par une lame d'air ayant une épaisseur égale à au moins 0,5 cm.

6. Dispositif selon l'une des revendications 4 ou 5, **caractérisé en ce que** l'axe dudit châssis (41) s'étend colinéairement à l'axe longitudinal dans un plan perpendiculaire audit élément de support (1).

7. Dispositif selon l'une quelconque des revendications 4 à 6, **caractérisé en ce que** ledit châssis (41) présente une section transversale dont la forme suit le profil de ladite couche chauffante (3a) sur ladite face interne de ladite couche chauffante.

8. Dispositif selon la revendication 7, **caractérisé en ce que** ladite section transversale dudit châssis s'étend sur une portion limitée dudit profil de ladite couche chauffante.

9. Dispositif selon l'une quelconque des revendications 4 à 8, **caractérisé en ce que** ledit châssis (41) comprend un plateau inférieur ajouré (42) comportant au moins une zone de réception (43, 44, 45) destinée à recevoir ledit photocatalyseur et une tôle supérieure ajourée fermant ledit châssis en regard de ladite face interne de ladite couche chauffante.

10. Dispositif selon l'une quelconque des revendications précédentes, **caractérisé en ce que** ledit logement de photocatalyseur intègre un tissu (6) apte à émettre des ondes électromagnétiques dans le domaine des infrarouges lointains dans sensiblement la même plage de longueurs d'onde que le rayonnement infrarouge émis par ladite couche chauffante.

## Patentansprüche

1. Vorrichtung zum Schwitzen durch Infrarot mit einem Trägerelement (1), das sich entlang einer Längsachse (X) erstreckt, das einen Benutzer in einer länglichen Position aufnehmen soll, und einem Deckelelement (2a, 2b) in einer halbzylindrischen Form, das an dem Trägerelement (1) montiert ist, um ein Innenvolumen zu begrenzen, das sich in der Längsrichtung des Trägerelements zwischen dem Trägerelement und der inneren Oberfläche des Deckelelements erstreckt, wobei die innere Oberfläche des Deckelelements zumindest teilweise mit einer Heizschicht (3a, 3b) bedeckt ist, die eine Strahlung im fernen Infrarot in zumindest einen Teil des Innenvolumens emittieren kann, wobei die Vorrichtung **dadurch gekennzeichnet ist, dass** sie eine Photokatalysatoraufnahme (4) umfasst, die für die emittierte Infrarotstrahlung durchlässig ist, einen Photokatalysator (5) abstützt und in der Nähe der inneren Fläche der Heizschicht (3a, 3b) angeordnet ist, um die Aktivierung des Photokatalysators mit Hilfe der durch die emittierte Infrarotstrahlung zugeführten Energie zu ermöglichen.

2. Vorrichtung nach Anspruch 1, **dadurch gekennzeichnet, dass** der Photokatalysator (5) ein photokatalytisches Produkt umfasst, das aus einem metallischen und/oder keramischen Substrat gebildet ist, auf dessen Oberfläche eine Titandioxidschicht ausgebildet ist.

3. Vorrichtung nach einem der vorangehenden Ansprüche, **dadurch gekennzeichnet, dass** die Heizschicht (3a, 3b) eine Infrarotstrahlung in einem Bereich von Wellenlängen zwischen 5 und 20 Mikrometer, vorzugsweise zwischen 8 und 14 Mikrometer, emittieren kann.

4. Vorrichtung nach einem der vorangehenden Ansprüche, **dadurch gekennzeichnet, dass** die Photokatalysatoraufnahme (4) ein Gestell (41) mit einer im Wesentlichen länglichen Form umfasst, das gemäß der Längsachse zwischen zwei inneren Enden des Deckelelements befestigt ist, so dass es gegenüber und im Abstand von der inneren Fläche der Heizschicht (3a) gehalten wird, die die innere Oberfläche des Deckelelements (2a) bedeckt.

5. Vorrichtung nach Anspruch 4, **dadurch gekennzeichnet, dass** das Gestell (41) im Abstand von der inneren Fläche der Heizschicht montiert ist, indem es durch einen Luftspalt mit einer Dicke gleich mindestens 0,5 cm getrennt ist.

6. Vorrichtung nach einem der Ansprüche 4 oder 5, **dadurch gekennzeichnet, dass** die Achse des Gestells (41) sich kollinear mit der Längsachse in einer zum Trägerelement (1) senkrechten Ebene erstreckt.

7. Vorrichtung nach einem der Ansprüche 4 bis 6, **dadurch gekennzeichnet, dass** das Gestell (41) einen Querschnitt aufweist, dessen Form dem Profil der Heizschicht (3a) an der inneren Fläche der Heizschicht folgt.

8. Vorrichtung nach Anspruch 7, **dadurch gekennzeichnet, dass** der Querschnitt des Gestells sich auf einem begrenzten Anteil des Profils der Heizschicht erstreckt.

9. Vorrichtung nach einem der Ansprüche 4 bis 8, **dadurch gekennzeichnet, dass** das Gestell (41) eine durchbrochene untere Platte (42) mit mindestens einer Aufnahmezone (43, 44, 45), die den Photokatalysator aufnehmen soll, und ein durchbrochenes oberes Blech, das das Gestell gegenüber der inneren Fläche der Heizschicht schließt, umfasst.

10. Vorrichtung nach einem der vorangehenden Ansprüche, **dadurch gekennzeichnet, dass** die Photokatalysatoraufnahme ein Gewebe (6) integriert, das elektromagnetische Wellen im Gebiet von fernem Infrarot in im Wesentlichen demselben Bereich von Wellenlängen wie die durch die Heizschicht emittierte Infrarotstrahlung emittieren kann.

## Claims

1. Infrared sudation device comprising a support element (1) extending along a longitudinal axis (X) intended to receive a user in lying position and a cover element (2a, 2b) of semi-cylindrical shape mounted on said support element (1) so as to delimit an internal volume extending in the longitudinal direction of said support element between said support element and the internal surface of said cover element, said internal surface of said cover element being covered at least in part with a heating layer (3a, 3b) able to emit far-infrared radiation in at least one portion of said internal volume, said device being **characterised in that** it comprises a photocatalyst housing (4), permeable to the infrared radiation emitted, supporting a photocatalyst (5) and being arranged in proximity to the internal face of said heating layer (3a, 3b) so as to allow said photocatalyst to be activated using the energy supplied by said infrared radiation emitted.

2. Device according to claim 1, **characterised in that** said photocatalyst (5) comprises a photocatalytic product formed from a metal and/or ceramic substrate on the surface of which is formed a layer of titanium dioxide.

3. Device according to either of the preceding claims, **characterised in that** said heating layer (3a, 3b) is able to emit infrared radiation in a range of wavelengths between 5 and 20 micrometres, preferably between 8 and 14 micrometres.

4. Device according to any of the preceding claims, **characterised in that** said photocatalyst housing (4) comprises a frame (41) of substantially elongate shape, fixed between two inside ends, along the longitudinal axis, of said cover element, in such a way as to be maintained facing and at a distance from said internal face of said heating layer (3a) covering the internal surface of said cover element (2a).

5. Device according to claim 4, **characterised in that** said frame (41) is mounted at a distance from said internal face of said heating layer by being separated by an air gap having a thickness of at least 0.5 cm.

6. Device according to either claim 4 or claim 5, **characterised in that** the axis of said frame (41) extends collinearly with the longitudinal axis in a plane perpendicular to said support element (1).

7. Device according to any of claims 4 to 6, **characterised in that** said frame (41) has a cross section the shape of which follows the profile of said heating layer (3a) on said internal face of said heating layer.

8. Device according to claim 7, **characterised in that** said cross section of said frame extends over a limited portion of said profile of said heating layer.

9. Device according to any of claims 4 to 8, **characterised in that** said frame (41) comprises a perforated lower plateau (42) comprising at least one reception zone (43, 44, 45) intended to receive said photocatalyst and a perforated upper sheet closing said frame facing said internal face of said heating layer.

10. Device as claimed in any preceding claim, **characterised in that** said photocatalyst housing integrates a fabric (6) able to emit electromagnetic waves in the far-infrared range in substantially the same range of wavelengths as the infrared radiation emitted by said heating layer.
